(19) Europäisches Patentamt / European Patent Office / Office européen des brevets

(11) **EP 4 208 274 B1**

(12) **EUROPEAN PATENT SPECIFICATION**

(45) Date of publication and mention of the grant of the patent:
**18.09.2024   Bulletin 2024/38**

(21) Application number: **21777414.0**

(22) Date of filing: **03.09.2021**

(51) International Patent Classification (IPC):
*A61K 8/368* (2006.01)        *A61Q 19/00* (2006.01)
*B01D 9/00* (2006.01)         *A23L 33/105* (2016.01)
*A61K 8/9789* (2017.01)       *A61K 36/185* (2006.01)
*B01D 11/02* (2006.01)        *B01D 11/04* (2006.01)

(52) Cooperative Patent Classification (CPC):
**B01D 9/0054; A23L 33/105; A61K 8/368;**
**A61K 8/9789; A61K 36/185; A61Q 19/00;**
**B01D 11/0288; B01D 11/0492;** A61K 2236/00;
A61K 2800/10; B01D 11/0211; B01D 11/0265

(86) International application number:
**PCT/EP2021/074345**

(87) International publication number:
**WO 2022/049232 (10.03.2022 Gazette 2022/10)**

(54) **METHOD OF OBTAINING THC-FREE CANNABINOID CONCENTRATE**

VERFAHREN ZUR HERSTELLUNG EINES THC-FREIES CANNABINOIDKONZENTRAT

PROCÉDÉ D'OBTENTION D'UN CONCENTRÉ DE CANNABINOÏDES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Validation States:
**MA**

(30) Priority:   **04.09.2020   IT 202000021055**

(43) Date of publication of application:
**12.07.2023   Bulletin 2023/28**

(73) Proprietor: **Herbolea Biotech S.p.A.**
**50019 Sesto Fiorentino (IT)**

(72) Inventors:
• **VENTURINI DEL GRECO, Giovanni**
  **50121 FIRENZE (IT)**

• **DECORTI, Deborha**
  **34071 Cormons (IT)**
• **MISURI, Livia**
  **50132 Firenze (IT)**
• **MARCHI, Andrea**
  **52028 Terranuova Bracciolini (IT)**
• **PIANTINI, Sara**
  **50019 Sesto Fiorentino (IT)**

(74) Representative: **Zaccaro, Elisabetta et al**
**Notarbartolo & Gervasi S.p.A.**
**Viale Achille Papa, 30**
**20149 Milano (IT)**

(56) References cited:
**WO-A1-2019/057994      WO-A1-2020/028991**
**WO-A1-2020/176806      WO-A2-2004/026857**
**US-A1- 2019 359 550**

**Description**

**FIELD OF THE INVENTION**

The invention relates to a method of obtaining a THC-free cannabinoid concentrate.

**BACKGROUND**

*Cannabis sativa* L. is a prolific, but not exclusive, producer of a diverse group of isoprenylated resorcinyl polyketides collectively known as cannabinoids (Hanus et al. 2016) nor cannabinoids from cannabis are the only lipid based exogenous compounds interacting with the endocannabinoid system. In the last few years, other plants have been found to produce cannabinoid-like compounds and several non-traditional cannabinoid plant natural products have been reported to act as cannabinoid receptor ligands. Cannabinoids can also be produced from yeast, fungus, or bacteria.

The endocannabinoid system consists of the endogenous cannabinoids (endocannabinoids), cannabinoid receptors and the enzymes that synthesise and degrade endocannabinoids. Many of the effects of cannabinoids and endocannabinoids are mediated by two G protein-coupled receptors (GPCRs), CB1 and CB2, although additional receptors may be involved. CB1 receptors are present in very high levels in several brain regions and in lower amounts in a more widespread fashion. These receptors mediate many of the psychoactive effects of cannabinoids. CB2 receptors have a more restricted distribution, being found in a number of immune cells and in a few neurones. Both CB1 and CB2 couple primarily to inhibitory G proteins and are subject to the same pharmacological influences as other GPCRs. Thus, partial agonism, functional selectivity and inverse agonism all play important roles in determining the cellular response to specific cannabinoid receptor ligands.

By interating with the endocannabinoid system, exogenous cannabinoids or terpenoids, such ones from cannabis, are used to reduce nausea and vomiting during chemotherapy, to improve appetite in people with HIV/AIDS, and to treat chronic pain and muscle spasms. Cannabis, its constituent cannabinoids, and terpenes are used to treat diseases or improve symptoms. Cannabinoids are synthesised in plants in their carboxylic acid forms. Cannabinoid acids, such as CBGA, THCA, CBDA, CBCA, and CBDVA short for cannabigerolic acid, tetrahydrocannabinolic acid, cannabidiolic acid, cannabichromenic acid and cannabidivarinic acid respectively, are precursors to their metabolites, the neutral forms CBG (cannabigerol), THC (tetrahydrocannabinol), the primary psychotropic cannabinoid found in cannabis, CBD (cannabidiol), CBC (cannabichromen) and CBDV (cannabidivarin). Neutral forms are obtained from acidic forms through decarboxylation.

Until recently, cannabinoid acids were not considered to be able to survive metabolism (i.e. inhalation by the lungs or digestion by the stomach and intestines and processing by the liver); nor were they considered to have any pharmacological activity in and of themselves (Jung et al 2007; Takeda et al 2008).

However, recent in vitro and animal research using extracted CBGA, THCA, CBDA or CBDVA revealed measurable actions on certain enzymes and receptor sites, suggesting some potential therapeutic effects for these cannabinoids and necessitating the elucidation and refinement of specific extraction techniques that preserve these particular acidic forms of these cannabinoids in order to provide material for further experimentation and research.

In particular, acidic forms of cannabinoids, such as CBDA or CBDVA, have shown to provide specific biological activites that can be useful to treat health diseases, in some cases even superior to their respective neutral forms (WO2017025712A1 - Use of cannabinoids in the treatment of epilepsy; WO/2019/012267 - use of cannabinoids in the treatment of a neurodegenerative disease or disorder).

Cannabigerolic acid (CBGA) is the acidic form of cannabigerol (CBG). It is a dihydroxybenzoic acid that is olivetolic acid in which the hydrogen at position 3 is substituted by a geranyl group. CBGA is the biosynthetic precursor to Delta(9)-tetrahydrocannabinol, the principal psychoactive constituent of the Cannabis plant.

CBGA may help diabetic patients combat some of the disease's complications and comorbidities like cardiovascular disease. CBGA was studied in vitro and found to greatly inhibit the enzyme aldose reductase, a major contributor of the oxidative stress that leads to heart and other problems. As expected, the results of the CBGA tests were highly dose dependent. Synthetic inhibitor medications have severe side effects for many patients, so a CBGA plant-derived drug is a promising prospect (Smeriglio et al., 2018).

Finally, researchers looked at cytotoxic effects of CBGA extracted from cannabis and found that not only did the CBGA kill colon cancer cells, but it hastened early cancer cell death and arrested the cancer cell cycle. While more research is definitely needed, the researchers were encouraged that CBGA may effectively target not only colon cancer cells but could also prevent the growth and proliferation of polyps. Left untreated, these polyps grow into carcinomas (Nallathambi, 2018).

Tetrahydrocannabinolic acid (THCA) is the precursor for THC produced by the plant, and is decarboxylated to THC with heat, light and time (for example by heating, smoking or cooking). Unlike THC, THCA is not associated with psychotropic effects in monkeys, mice or dogs, and since we know these effects are due to CB1 receptor activation, this suggests that THCA is not a strong activator of this receptor.

Cannabidiolic acid (CBDA) is the precursor for CBD produced by the plant that is decarboxylated to CBD with heat, light and time. There is a limited amount of research on CBDA, the majority of which has been on the anti-nausea effects of CBDA. Like CBD, CBDA suppresses nausea and vomiting in rats and shrews through the serotonin receptor (5HT1A), and could decrease intestinal motility, suggesting a role for CBDA in regulating nausea, for example in patients undergoing chemotherapy (Bolognini et al 2013). Like CBD, CBDA has also been shown to reduce stress in rats, again through the serotonin receptor. Other pharmacology targets of CBDA that have been identified include inhibition of enzymes in the endocannabinoid system, TRPV1 activation and cyclooxygenase (COX) inhibition. CBDA appears in vivo and in vitro to work pharmacologically more similarly to CBD (e.g. both via serotonin-receptor activation), though CBDA was shown to be more potent than CBD in its serotonin-receptor-mediated effects. Additionally, CBDA has been shown in vitro to block, in varying degrees, both cyclooxygenase (COX) enzymes 1 and 2, which are each distinct mediator of inflammation and pain secondary to inflammation. Non-steroidal antiinflammatory (NSAID) drugs such as acetylsalicylic acid (aspirin), ibuprofen, naproxen, indomethacin, and diclofenac all work via COX 1 and 2 inhibition, and, like CBDA, contain a carboxylic acid group in their structures that suggests this part of the molecule is integral to the way they work.

In one assay, CBDA significantly inhibited both COX 1 and 2-mediated oxidation activity, with the CBDA showing a strong preference for inhibiting COX 2 specifically (Takeda et al. 2008). A second study demonstrated that CBDA inhibited COX 1 significantly but only THCA inhibited COX 2, and by only a little over 30% (Ruhaak, L. et al 2011). Both studies showed that the carboxylic acid forms CBDA and THCA had stronger overall COX-inhibiting activity than their de-carboxylated forms CBD and THC, however.

Lastly, CBDA showed in vitro activity at some of the various cation channel receptors collectively known as transient receptor potentials that play important roles in pain and inflammation signal transduction such as TRPV1 and TRPV4 (the "vanilloid" type); TRPA1 (the "ankyrin" type) and TRPM8 (the "melastatin" type). They can block, activate, or de-sensitize these to activation by another activator (Cascio and Pertwee 2014). These are likely additional mechanisms by which the carboxylic acid forms of the cannabinoids work independently of their de-carboxylated forms to moderate pain and inflammation both centrally and peripherally.

To facilitate the manufacturing of various products that could be safely administered to and consumed by patients and/or consumers, cannabinoids are usually extracted from the biomass, concentrated, and purified to obtain various concentrates or isolates. However, typical methods to extract neutral cannabinoids are not ideal to extract cannabinoid acids and present several limitations.

In some applications, cannabinoid acids present favorable chemical-physical features properties over neutral forms. For vaping application, for instance, the possibility to utilize concentrates having a high content of CBDA, in place of CBD, is helpful to avoid the formation of crystals in the vaping cartridges.

Cannabinoid concentrates can be produced through several techniques. Typically, they are obtained from biomass that has been previously dried by means of supercritical fluid extraction (SFE), as with supercritical $CO_2$, followed by a winterization step to remove chlorophyll and waxes. Winterization encompasses the use of ethanol or butane at low temperatures (US 9186386 B2, US 6403126 B1). Such process presents several drawbacks such as the high investment required, the need for highly skilled technicians to utilize complex equipment, the use of flammable and harmful organic solvents to winterize the crude extract, the high energy consumption.

It is challenging to completely remove organic solvents used in combination with $CO_2$ during the extraction step or to remove chlorophyll in the winterization step. The technical challenge to overcome has led policymakers to set content limits for organic solvents, some of which are known cancerogenic compounds, as high as 5.000 ppm (source Health Canada). Additionally, supercritical $CO_2$ has high selectivity for toxic components which might be present in pesticides, therefore a risk associated to their presence in concentrated form in the final product might be present. Furthermore, as heat is required to dry the biomass and remove the solvents as well as it is generated through the $CO_2$ extraction step, it is difficult to well preserve heat-sensitive acidic forms that can decarboxylate. The cannabinoids content achieved with such process is not sufficiently high to go directly into a crystallization step. An intermediate distillation step is often required. Finally, supercritical $CO_2$ cannot extract with the same efficiency acidic forms of cannabinoids due to higher molecular weight compared to the neutral forms. All these aspects make the whole process not an ideal option to extract and concentrate acidic forms of cannabinoids.

A more recent alternative technique is represented by cryogenic-ethanol, a process in which a biomass that has been previously dried is extracted at very low temperatures (-40°C) to avoid extraction of chlorophyll and waxes into the solvent. The cannabinoids-enriched ethanol solution is then evaporated to recover the solvent. Such activity is energy intensive and it can be very time consuming, considering the large volumes of solvents to be evaporated (up to 20 times biomass weight). During such step is challenging to avoid decarboxylation of cannabioind acids. Finally, the use of organic solvents inherently results in safety, health and environmental issues.

WO2019057994A1 describes a method for the preparation of a whole plant extract comprising at least 50 w/w% cannabidiol (CBD) and less than 0.2 w/w% tetrahydrocannabinol (THC) from cannabis plant material, comprising the steps of:

a) providing cannabis plant material comprising cannabinoids and acids thereof,

b) incubation of the plant material of step a) in an aqueous medium comprising salt and/or hydroxide, at 25 - 75 °C in a medium comprising between 30 to 100 v/v% alcohol for 0.5 to 1 hour, to allow conversion of cannabinoid acids and cannabinoids into the respective cannabinoid salts and/or complexes thereof,

c) allowing the cannabinoid salts and/or complexes of step b) to separated from waxes contained in the plant material, resulting in aqueous cannabinoid salts or complexes on a surface of raw material,

d) mixing the aqueous cannabinoid salts or complexes of step c) with an alcohol, resulting in an alcoholic cannabinoid extract,

e) bringing the water to alcohol ratio of the alcoholic extract of step c) to 30 - 70 : 70 - 30 v/v% providing an aqueous extract comprising the cannabinoid salts and complexes,

f) acidification of the aqueous extract of step e) resulting in the cannabinoid salts and complexes to be converted into their acids, producing an aqueous acid extract,

g) subjecting the cannabinoid acids of step f) to decarboxylation with basic $Al_2O_3$ at a temperature of at least 20 °C resulting in preferential formation of THC as compared to formation of CBD, providing a decarboxylated mixture,

h) optionally, further incubating the decarboxylated mixture of step g) with basic $Al_2O_3$ at a temperature of at least 20 °C to convert the cannabinoid acids and the cannabinoids into cannabinoid salt and/or complexes, respectively,

i) adding alcohol to the mixture of step h) to a water-alcohol ratio of 30 - 70:70 - 30 wherein the THC dissolves while the cannabinoid salts and complexes do not dissolve,

j) contacting the mixture of step i) to an adsorbent or absorbent, allowing the THC to eliminate from said adsorbent or absorbent, resulting in a THC depleted mixture,

k) acidification of the THC depleted mixture of step j) resulting in conversion of the cannabinoid salts and complexes in the THC depleted mixture, into cannabinoid acids, and removing of said mixture from the adsorbent or absorbent with an alcohol,

l) decarboxylation of the cannabinoid acids into their respective cannabinoids at a temperature in the range of 50 to 150 °C, and vacuum in the range of 0 to 1000 mBar to achieve a desired decarboxylation degree between 1 to 10 hours, resulting in a decarboxylated, THC depleted mixture,

m) recovery of one or more of cannabinoids from the decarboxylated THC depleted mixture of step l).

The Applicant noted in particular that the method comprises the use of organic solvents on plant material, the decarboxylation of acidic cannabinoids in presence of $Al_2O_3$ to preferentially form THC, and the filtration of the alcoholic solution.

US2019359550 describes a method to recover acidic cannabinoids by leaching plant material or lipophilic extracts such as Butane Hash Oil - BHO after winterization. The Applicant notes that the method results in a microcrystalline powder indistinctly rich in all cannabinoid acids that are present in the starting lipohilic extract by utilizing a pH of of at least 8.8, and that the method does not teach how to obtain a concentrate depleted in THCA while rich in other cannabinoid acids, e.g. CBDA or CBGA, unless chromatography is considereded as a post-refining step. In particular, the Applicant notes that the method results in a high THCA content concentrate, having a THCA content higher than the starting lipophilic extract, and that the method does not teach how to obtain a cannabinoid concentrate low in THCA content, wherein the THCA content is lower than the THCA content of the starting lipophilic extract. Furthermore, the Applicant notes that the method requires the use of harmful solvents (i.e. buthane or ethanol). Additionally, the Applicant notes that US2019359550 specifies that the leaching step is performed for 20 min. Finally, the Applicant notes that the method does not allow to obtain a concentrate rich in CBDA or CBGA without concentrating THCA in any steps of the manufacturing process.

US7592468B2 describes a method of production of THC comprising extracting THC and THC carboxylic acid from plant material using a first solvent, extracting the acid using a second solvent, wherein the solvent preferentially dissolves the acid compared to THC, converting the THC acid into a derivative and extracting the derivative into a third solvent.

US9340475B2 teaches a method to decarboxylate CBDA in hemp oil, followed by distillation of CBD from the decarboxylated hemp oil, THC conversion to CBN, winterization with isopropanol and, finally, silica plug eluted with exane-ethyl acetate to remove impurities. WO 2004/026857 A2 discloses a method of obtaining a pure cannabinoid from a plant material and WO 2020/028991 discloses a lipid extraction process avoiding the use of harmful solvents in the synthesis of cannabis extract.

As a precursor of THC and regardless lacking psychotropic activity, THCA is often subject to same content limitation as THC. Therefore, concentrates tipycally must be further refined to remove or reduce THCA and THC to regulatory compliant levels, e.g. 0.3% weight by weight.

In some cases, THC and/or THCA concentration levels must remain compliant with regulatory content levels at any time during the manufacturing process. For instance in the US, the federal law requires manufacturers willing to produce CBD concentrates not to trespass the threshold of 0.3% total THC content w/w in any steps of the manufacturing process. Current extraction techniques, such as CO2 or cryo-ethanol, obtain an intermediate product that has a THC content higher than the allowed content of 0.3% w/w by means of chromatography or crystallization techniques.

Chromatography can be a very time consuming and costly process and presents some limitations in scaling-up. Furthermore, chromatographic purification methods such as flash chromatography can have a high environmental impact since they typically involve large quantities of harmful or toxic solvents run at high flow rates.

Crystallization also involves the use of large volumes of organic solvents, such as pentane or eptane, making this step dangerous and not environmentally friendly.

## SUMMARY OF INVENTION

The Applicant noted that, even if methods for obtaining THCA- or THC-free cannabinoid acids concentrates are known, they result in very long and expensive operations that present several limits and need still to be improved, in particular in terms of preservation of cannabinoid acids, cannabinoid concentration, efficiency, cost-effectiveness, environmental impact, utilization of toxic or harmful solvents, presence of residual organic solvents in products, THC and/or THCA concentration levels compliant with regulatory frameworks throughout the manufacturing process.

For example, the Applicant noted that, even if WO 2018/130682 provides a novel and environmentally friendly method of enzyme-assisted lipid-based extraction showing a remarkable efficiency in extracting and stabilizing cannabinoids, even in their original acidic forms, such method presents some limitations in obtaining concentrates (>40% cannabinoids content), especially starting from low cannabinoids content material, such as hemp biomass. Furthermore, such method does not allow a selective separation of the acidic forms from the neutral forms in the lipid extract. Finally, such method does not allow to obtain a THCA- or THC-free cannabinoid concentrate.

The Applicant also noted that purification techniques commonly used to purify cannabinoids concentrates typically apply extracting, concentrating, and purifying techniques that result in a decarboxylation of cannabinoid acids and the use of organic solvents.

Hence, the Applicant felt that a simpler way to obtain THCA- or THC-free cannabinoids concentrates, even in their acidic forms, would therefore be desirable and that a process that could efficiently generate such concentrates, preserving a high level of cannabinoid acids, without making use of any organic solvent or costy techniques, such as chromatography, to remove THC or THCA, would represent a healthier and safer process for workers and consumers as well as a more environmentally friendly and convenient solution.

An object of the present invention is therefore the provision of method for preparing a cannabinoid concentrate making no use of organic solvents, wherein the content of THC and THCA is significantly reduced or brought to undetectable level, capable of attaining a high concentration of cannabinoids, while preserving cannabinoid acids, other than THC and THCA, that is efficient, cost-effective, environmentally friendly, even when starting from low cannabinoids content material such as hemp biomass.

Therefore, the present invention relates, in a first aspect, to a method for preparing a cannabinoid concentrate comprising more than 50% by dry weight, with respect to the total dry weight of the concentrate, of cannabinoids and wherein the weight ratio (THCA):(other cannabinoid acids) is of less than 1:50, comprising the steps of:

a) providing a lipid extract comprising at least 1% by dry weight, with respect to the total cannabinoids dry weight, of cannabinoid acids and wherein the weight ratio (THCA):(other cannabinoid acids) is of less than 1:2;

b) mixing said lipid extract with an alkaline aqueous solution to form a mixture having a pH of at least 12;

c) separating from the mixture of step b) an aqueous phase containing cannabinoid salts; and

d) obtaining said cannabinoid concentrate, wherein before step c) at least one salt and/or at least one sugar is added to the mixture of step b), and said step c) of separating comprises separating from the mixture of step b):

- a lighter oily phase,
- an intermediate slimy phase, and
- a heavier aqueous phase, wherein the heavier aqueous phase is the aqueous phase containing cannabinoid acid salts.

Surprisingly, the Applicant has found out that by applying the described method is possible to obtain cannabinoid products having not only i) a higher content of cannabinoids and ii) a higher content of cannabinoid acids, but also iii) a lower THCA and/or THC content, and iv) a decreased ratio (THCA):(other cannabinoids), compared to the starting lipid extract, making no use of organic solvents.

Furthermore, the Applicant has found out that by applying the described method is possible to obtain cannabinoid concentrates without exceeding a THCA content of 0.3% w/w in any intermediates of any steps of the manufacturing process, in full compliance with regulatory requirements.

Thanks to the specific conditions of the method according to the invention, a cannabinoid concentrate is indeed obtained, showing an unexpectedly high level of preservation of cannabinoid acids other than THCA.

The above method produces a concentrate comprising more than 50% by weight, with respect to the total dry weight of the concentrate, of cannabinoid acids and not more than 0.3% by weight, with respect to the total dry weight of the concentrate, of total THC.

The above method produces a cannabinoid concentrate comprising more than 50% by weight, with respect to the total dry weight of the concentrate, of cannabinoid acids and not more than 0.0001 % per cent by weight of one or more organic solvents selected from a group consisting of acetone, benzene, butane, chloroform, cyclohexane, dichloromethane, ethanol, ethyl acetate, ethylbenzene, heptane, hexane, isobutane, isopropanol, methanol, pentane, propane, toluene, m-xylene, o-xylene, and p-xyleneheptane.

The advantages of these cannabinoid concentrates have been disclosed in relation to the method according to the first aspect of the present invention and are not herewith repeated.

Thanks to its compositional and purity properties, said cannabinoid concentrate may be advantageously used for preparing pharmaceutical or nutraceutical products, cosmetics, food or feed products, antimicrobial, antibacterial, insecticidal or biopesticides containing one or more cannabinoids.

In a further aspect, therefore, the present invention relates to a method for preparing a pharmaceutical product, a nutraceutical product, a cosmetic product, a food product, a feed product, an antimicrobial, an antibacterial, an insecticide, a biopesticide, comprising the step of:

- providing a cannabinoid concentrate obtained using the method according to the present invention and/or preparing a cannabinoid concentrate according to the present invention; and
- obtaining a pharmaceutical product, a nutraceutical product, a cosmetic product, a food product, a feed product, an antimicrobial, an antibacterial, an insecticide, a biopesticide comprising one or more cannabinoids.

## BRIEF DESCRIPTION OF THE DRAWINGS

Figure 1 shows the HPLC trace of the lipid extract of Example 1;
Figure 2 shows the HPLC trace of the lighter oily phase obtained from the separation step of Example 1;
Figure 3 shows the HPLC trace of the intermediate slimy phase obtained from the separation step of Example 1; and
Figure 4 shows th the HPLC trace of the precipitate obtained from the heavier aqueous phase obtained from the separation step of Example 1.

## DETAILED DESCRIPTION OF THE INVENTION

The present invention relates, in a first aspect, to a method for preparing a cannabinoid concentrate comprising more than 50% by dry weight, with respect to the total dry weight of the concentrate, of cannabinoids and wherein the weight ratio (THCA):(other cannabinoid acids) is of less than 1:50, comprising the steps of:

a) providing a lipid extract comprising at least 1% by dry weight, with respect to the total cannabinoids dry weight, of cannabinoid acids and wherein the weight ratio (THCA):(other cannabinoid acids) is of less than 1:2;
b) mixing said lipid extract with an alkaline aqueous solution to form a mixture having a pH of at least 12;
c) separating from the mixture of step b) an aqueous phase containing cannabinoid salts; and
d) obtaining said cannabinoid concentrate, wherein before step c) at least one salt and/or at least one sugar is added to the mixture of step b), and said step c) of separating comprises separating from the mixture of step b):

- a lighter oily phase,
- an intermediate slimy phase, and
- a heavier aqueous phase, wherein the heavier aqueous phase is the aqueous phase containing cannabinoid acid salts.

Surprisingly, the Applicant has found out that by applying the described method is possible to obtain cannabinoid products having not only i) a higher content of cannabinoids and ii) a higher content of cannabinoid acids, but also iii) a lower total THC content, and iv) a decreased ratio (THCA):(other cannabinoids), compared to the starting lipid extract, making no use of organic solvents.

Furthermore, the Applicant has found out that by applying the described method is possible to obtain cannabinoid concentrates without exceeding a THCA content of 0.3% w/w in any intermediates of any steps of the manufacturing process, in full compliance with regulatory requirements.

Within the framework of the present description and in the subsequent claims, except where otherwise indicated, all the numerical entities expressing amounts, parameters, percentages, and so forth, are to be understood as being preceded in all instances by the term "about". Also, all ranges of numerical entities include all the possible combinations of the maximum and minimum values and include all the possible intermediate ranges, in addition to those specifically indicated herein below.

Listed below are definitions of various terms used to describe this invention. These definitions apply to the terms as they are used throughout this specification and claims, unless otherwise limited in specific instances, either individually or as part of a larger group.

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and peptide chemistry are those well-known and commonly employed in the art.

As used herein, the articles "a" and "an" refer to one or to more than one (i.e. to at least one) of the grammatical object of the article. By way of example, "an element" means one element or more than one element. Furthermore, use of the term "including" as well as other forms, such as "include", "includes," and "included," is not limiting.

As used herein, the term "cannabinoid" includes, but is not limited to, cannabinol (CBN), cannabinolic acid (CBNA), $\Delta(9)$-tetrahydrocannabinol ($\Delta(9)$-THC), $\Delta(9)$-tetrahydrocannabinolic acid ($\Delta(9)$-THCA), cannabidiol (CBD), cannabidiolic acid (CBDA), $\Delta(8)$-tetrahydrocannabinol ($\Delta(8)$-THC), $\Delta(8)$-tetrahydrocannabinolic acid ($\Delta(8)$-THCA), cannabivarin (CBV), cannabivarinic acid, cannabigerol (CBG), cannabigerolic acid (CBGA), cannabichromene (CBC), cannabichromenic acid (CBCA), cannabicyclol (CBL), cannabicyclolic acid (CBLA), Cannabidivarin (CBDV) and cannabidivarin acid (CBDVA).

As used herein, with the expression "THC" is meant tetrahydrocannabinol, encompassing its isomeric forms $\Delta(9)$-tetrahydrocannabinol ($\Delta(9)$-THC) and $\Delta(8)$-tetrahydrocannabinol ($\Delta(8)$-THC).

As used herein, with the expression "CBD" is meant cannabidiol.

As used herein, with the expression "THCA" is meant tetrahydrocannabinolic acid, encompassing its isomeric forms $\Delta(9)$-tetrahydrocannabinolic acid ($\Delta(9)$-THCA) and $\Delta(8)$-tetrahydrocannabinolic acid ($\Delta(8)$-THCA).

As used herein, with the expression "CBDA" is meant cannabidiolic acid.

As used herein, the term "cannabinoid acids" includes, but is not limited to, cannabidiolic acid (CBDA), cannabinolic acid (CBNA), cannabigerolic acid (CBGA), cannabichromenic acid (CBCA), cannabicyclolic acid (CBLA), cannabidivarinic acid (CBDVA), cannabigerovarinic acid (CBGVA), tetrahydrocanabivarinic acid (THCVA), cannabichromevarinic acid (CBCVA), cannabidiphorol acid (CBDPA) and $\Delta9$-tetrahydrocannabiphorol acid (THCPA).

As used herein, the term "total THC" is equal to the sum of THCA content multiplied by the molecular weight ratio 0.877 plus THC content, as per the formula:

$$\text{total THC} = \text{THCA} \times 0.877 + \text{THC}.$$

As used herein, the term "terpenes" includes, but is not limited to, pinene, limonene, $\alpha$-terpinene, terpinen-4-ol, carvacrol, carvone, 1,8-cineole, p-cymene, fenchone, $\beta$-myrcene, cannaflavin A, cannaflavin B, nerolidol, phytol and squalene.

As used herein, the term "lipids" includes, but is not limited to, olive oil, coconut oil, vegetable oil, milk, butter, liposomes, glycerine, polyethylene glycol, ethyl acetate, d-limonene, liquid paraffin, butylene glycol, propylene glycol, ethylhexyl palmitate.

As used herein, the term "about" will be understood by persons of ordinary skill in the art and will vary to some extent on the context in which it is used. As used herein when referring to a measurable value such as an amount, a temporal duration, and the like, the term "about" is meant to encompass variations of $\pm20\%$ or $\pm10\%$, including $\pm5\%$,

±1%, and ±0.1% from the specified value, as such variations are appropriate to perform the disclosed methods.

The present invention may present in one or more of the above aspects one or more of the characteristics disclosed hereinafter.

Further features and advantages of the invention will appear more clearly from the following description of some preferred embodiments thereof, made hereinafter by way of a non-limiting example with reference to the following exemplary examples.

In a preferred embodiment, the content of total THC does not exceed 0.3% by dry weight in any stream in any step of the method according to the present invention. In other words, none of the solutions, phases, extracts, or other streams used or obtainable according to the method contains more than 0.3% by dry weight of total THC.

The method according to the present invention comprises the step a) of providing a lipid extract comprising at least 1% by dry weight, with respect to the total cannabinoids dry weight, of cannabinoid acids and wherein the weight ratio (THCA):(other cannabinoid acids) is of less than 1:2.

Preferably, said lipid extract of step a) comprises no more than 1% by dry weight of THCA.

Preferably, said lipid extract of step a) comprises at least 20% by dry weight, on total cannabinoids weight, of cannabinoid acids.

Preferably, said lipid extract has a total cannabinoids content of at least 2% by dry weight, more preferably of at least 3% by dry weight, even more preferably of at least 5% by dry weight.

Preferably, said lipid extract has a cannabinoid acids content of at least 1% by dry weight, more preferably of at least 2% by weight, even more preferably of at least 3% by dry weight, wherein said cannabinoid acids are more preferably selected from the group consisting of cannabigerolic acid (CBGA) and tetrahydrocannabidiolic acid (CBDA).

Preferably, the lipid of said lipid extract of step a) is selected from the group consisting of: vegetable oil, milk, butter, liposomes, ethyl acetate, glycerine, d-limonene, caryophyllene, liquid paraffin, mineral oil, paraffin wax, microcrystalline wax, mineral wax, ozokerite, polyethylene, polyoxyethylene and hydrocarbon waxes derived from carbon monoxide and hydrogen, cerosin; cetyl esters; hydrogenated joioba oil, butylene glycol, propylene glycol, polyethylene glycol, liposomes, lecithin, ethylhexyl palmitate, or mixtures thereof.

In another embodiment, the lipid is a vegetable oil.

Preferably, said vegetable oil is selected from the group consisting of: olive oil, coconut oil, sunflower oil, sesame oil, hemp seed oil, Medium-chain triglycerides (MCTs) oil, or a mixture thereof.

In an embodiment, the lipid is olive oil. In another embodiment, the lipid is coconut oil. In yet another embodiment, the lipid is sunflower oil. In another embodiment, the lipid is Medium Tryglicerids Chains (MCTs) oil. In an embodiment, the lipid is milk. In a further embodiment, the lipid is butter. In yet another embodiment, the lipid is a liquid paraffin.

Preferably, the lipid is a vegetable oil having a free-fatty acids (FFA) content below 1%, preferably below 0.5%, even more preferably below 0.1 %.

In a preferred embodiment of the method according to the present invention, the lipid extract of step a) containing cannabinoids is obtained from a biological material containing cannabinoids, preferably chosen from the group consisting of a plant, an alga, a bacterium, a yeast, a fungus, a genetically engineered micro-organism, or a mixture thereof.

That is, the method according to the invention preferably comprises a step of obtaining a lipid extract containing cannabinoids from a biological material containing cannabinoids, preferably chosen from the group consisting of a plant, an alga, a bacterium, a yeast, a fungus, a genetically engineered micro-organism, or a mixture thereof.

In an even more preferable embodiment, the lipid extract of step a) containing cannabinoids is obtained by putting in contact a biological material containing cannabinoids with a vegetable oil.

In an embodiment the initial lipid extract is obtained by mixing a lipid with a cannabinoid extract obtained by means of supercritical $CO_2$ extraction or by means of organic solvents.

Preferably, the lipid extract of step a) is obtained from a biological material containing cannabinoids by means of the steps of:

I. comminuting a biological material containing cannabinoids;

II. mixing the comminuted biological material with lipids to obtain a mixture;

III. agitating the mixture at a temperature range of 1 to 80 °C; and

IV Obtaining a lipid extract from the mixture of step III.

Preferably, said biological material containing cannabinoids is selected from a plant, an alga, a bacterium, a yeast, a fungus, a genetically engineered micro-organism, or a mixture thereof.

In said step I., the biological material is comminuted to increase the surface contact. Then water, lipids are added to the plant material to form a homogeneous mixture or slurry; the mixture may be agitated through stirring or other agitation methods preferably for at least 30 min. Ultrasound/sonication or microwaves or steam explosion may advantageously be used before or after adding lipids to the mixture to reduce the time necessary to achieve biological material dissolution and high cannabinoids lipid-extraction yield.

The mixture obtained is then separated for example via density separation (i.e. centrifugation) or pressing (French press) and/or filtration to recover a lipid fraction highly enriched with cannabinoids and waxes free.

In said preferred embodiment, steps I. and II. may be also inverted.

Preferably, said biological material containing cannabinoids is selected from the Cannabis *genus* of plants, wherein said biological material is pure, a hybrid or genetically modified variant thereof. Preferably, said biological material containing cannabinoids selected from the Cannabis *genus* of plants, belongs to the *species* C. sativa (hemp), C. indica and C. ruderalis.

Preferably, said biological material containing cannabinoids is industrial hemp of the species C. sativa. In the context of the present invention, preferred cannabis plant material is fibre hemp or industrial hemp, in particular of the following strains: Fedora 17, Felina 34, Ferimon 12, Futura 75, Carmagnola, Santhica 70, inter alia with relatively high content of CBDA in % by weight.

Preferably, the biological material containing cannabinoids has a moisture content of at least 20% of the biological material weight.

Preferably, said biological material containing cannabinoids is newly harvested and has a moisture content of at least 30%, preferably at least 40%.

Preferably, said biological material can be used in said step I. of the method according to the invention either fresh or dried. In an embodiment, the biological material is newly harvested and contain high level of moisture; in such a case addition of extra water to the biological material is unnecessary.

Preferably, the biological material containing cannabinoids has a total cannabinoid content of at least 0.1% by weight, more preferably of at least 0.2 % by weight, even more preferably of at least 1% by weight, even more preferably of at least 2% by weight.

Preferably, said biological material contains at least 0.5% cannabinoids in weight.

Preferably, the biological material containing cannabinoids is industrial hemp comprising less than 0.6% by weight of total THC, more preferably less than 0.2% by weight of total THC, or is cannabis comprising more than 0.2% by weight of total THC, more preferably more than 0.6% by weight of total THC, or hybrids and genetically modified variants thereof.

In a preferred aspect, said biological material is chosen from the group consisting of buds, flowers, leaves, stalks, stems, roots and seeds or a mixture thereof. In an embodiment, the biological material includes seeds. In another embodiment, when the biological material includes seeds, no lipid is added. In a further embodiment, when the biological material includes seeds, a lipid is added. Biological material including seeds may be rich in lipids, and thus may not need the further addition of lipids.

Preferably, in step II. the lipids have been neutralized prior being added to the mixture.

Preferably, enzymes are added to the mixture of said step II.

Preferably, said enzymes are one or more independently selected from the group consisting of oxidoreductases, transferases, hydrolases, lyases, isomerases, and ligases, cellulase, hemicellulase, xylanase, glucanase, beta-glucanase, pectinase, glucuronyltransferase, lipase, amylase, alpha-amylase, beta-amylase, phospholipase, arabanase, galacto-, beta-mannanase, protease, phytase, cannabinoid synthase, THC synthase, CBD synthase, CBCA synthase.

In an embodiment, said enzyme is cellulase. In another embodiment, said enzyme is beta-glucosidase. In another embodiment, said enzyme is hemicellulase. In another embodiment, said enzyme is xylanase. In yet another embodiment, said enzyme is glucanase. In yet another embodiment, said enzyme is pectinase. In still another embodiment, said enzyme is amylase. In yet another embodiment, said enzyme is lipase or phospholipase. In said another embodiment, said enzyme is glucuronosyltransferase or alcohol dehydrogenase. In yet another embodiment, said enzyme is arabinanase. In still another embodiment, said enzyme is phytase. In a further embodiment, said enzyme is protease. In yet another embodiment, said enzymes is a cannabinoid synthase. In still another embodiment, said enzyme is THCA synthase. In yet another embodiment said embodiment is CBDA synthase. In another embodiment, said enzyme is CBCA synthase.

Preferably, in step II. the temperature varies in the range from 40 to 70 °C. Preferably, in step II. the pH varies in the range from 4 to 6.

Preferably, the aqueous phase of said step III. is degummed with phosphoric acid.

Preferably, said step IV comprises separating the mixture into a lipid phase, an aqueous phase, and a solid phase; wherein the lipid phase comprises the lipid extract.

In an embodiment, in step IV. the mixture is separated by density. In a further embodiment, in step IV. the mixture is separated by pressing and/or filtering.

In a further embodiment, in step IV.the mixture is separated into a lipid-soluble phase and a wet solid phase.

In an embodiment, the lipid-soluble extract is recirculated any number of times to achieve higher cannabinoid or terpene content.

In an embodiment, the lipid-soluble extract is recirculated any number of times to achieve higher cannabinoid content.

In a further embodiment, at least 50%, preferably 70% of the terpenoids, at least 70% of the diterpenoids and at least 50%, preferably 70% of monoterpenes contained in the plant material are extracted into the lipid-soluble extract.

In a still further embodiment at least 70% of the sesquiterpenes and at least 50% of the mono-terpenes contained in the plant material are extracted into the lipid-soluble extract.

In an embodiment, the lipid-soluble extract has a total cannabinoid content of at least 2% by weight. In a further embodiment, the lipid-soluble extract has a total cannabinoid content of at least 3% by weight. In yet another embodiment, the lipid-soluble extract has a total cannabinoid content of at least 5% by weight.

In an embodiment, the two main cannabinoids in the lipid-soluble extract are preferably CBGA and CBDA, or any cannabinoid other than THCA.

Preferably, less than 10%, preferably less than 5%, more preferably less than 2%, of cannabinoids are decarboxylated during said steps I.-IV. of obtaining the lipid extract containing cannabinoids from a biological material containing cannabinoids.

In an embodiment the lipid extract of step a) is not winterized before mixing with alkaline solution of step b). In a preferred embodiment of the method according to the present invention, the aqueous phase resulting from said step of separating the mixture into a lipid phase, an aqueous phase, and a solid phase, wherein the lipid phase comprises the lipid extract, can also be used in the production of nutraceutical, antimicrobial, antibacterial products or biopesticides.

The method according to the present invention comprises a step b) of mixing said lipid extract with an alkaline aqueous solution to form a mixture having a pH of at least 12.

Preferably, the alkaline aqueous solution of step b) comprises at least one hydroxide of at least one metal selected from the group consisting of: alkali metal, and alkaline earth metal.

Preferably, the alkaline aqueous solution of step b) comprises NaOH, KOH or a mixture thereof.

Preferably, the alkaline aqueous solution of step b) is 0.5 M NaOH or KOH.

More preferably, the alkaline aqueous solution of step b) is 0.1 M NaOH or KOH.

Preferably, in step b) the alkaline aqueous solution is added to the lipid extract in a weight ratio (alkaline aqueous solution):(lipid extract) of at least 2:1, more preferably of at least 3:1, even more preferably of at least 4:1.

Preferably, in step b) the alkaline aqueous solution has a molarity of NaOH calculated on the base of the total acidity of the lipid extract expressed as moles of KOH required for acidic tritation of the lipid extract, so that the ratio NaOH mol / KOH mol is in the range of from 5 to12, even more preferably from 7 to10.

Preferably, the mixture of step b) is mixed at 25 °C for a time in the range from at least 5 seconds to less than 60 minutes, more preferably from 5 to 20 minutes, even more preferably from 5 to 10 min. In case the mixing is performed in continuous, the contact time between the lipid extract and the alkaline solution is preferably less than 5 minutes.

Preferably, the mixture of step b) has a pH value ranging from 12.6 to 13.5, optimally about 13.

The method according to the invention comprises a step c) of separating from the mixture of step b) an aqueous phase containing cannabinoid salts.

Before step c) at least one salt and/or at least one sugar is added to the mixture of step b).

By adding at least one salt and/or at least one sugar to the mixture of step b), an increase of the density of the aqueous phase is obtained, thus facilitating its separation in step c).

Preferably, said at least one salt is selected from the group consisting of: sodium chloride (NaCl), and calcium chloride ($CaCl_2$).

Preferably said at least one sugar is selected from the group consisting of: glucose and fructose.

In a preferred embodiment, said at least one salt is sodium chloride.

Preferably said at least one salt is calcium chloride.

Advantageously, when said at least one salt is calcium chloride, said salt may be subsequently removed after separation of the aqueous phase in step c).

In the method according to the present invention, step c) of separating comprises separating from the mixture of step b):

- a lighter oily phase,
- an intermediate slimy phase, and
- a heavier aqueous phase, wherein the heavier aqueous phase is the aqueous phase containing cannabinoid acid salts.

In this way, three phases are advantageously obtained. The lighter oily phase contains neutral cannabinoids and most of THCA, but is depleted of cannabinoid acids other than THCA, the intermediate slimy phase comprises impurities and a remaining amount of THCA, whereas the heavier aqueous phase contains cannabinoid acid salts, which in view of the ripartition of THCA in this three phases system, are different from the salt of THCA.

Preferably, said lighter oily phase depleted of cannabinoid acids is distilled to recover decarboxylated cannabinoids and/or terpenes.

In this way, a fraction suitable as component of broad-spectrum oils may be advantageously obtained.

The method according to the invention comprises a step d) of obtaining said cannabinoid concentrate.

Preferably, said step d) comprises filtering said aqueous phase of said step c).

Preferably, said filtering is carried out with a fiberglass filter or a paper filter.

Preferably, said filtering is carried out with a filter having pores diameter of less than 2.5 microns, more preferably of less than 2 microns, even more in the range from 0.5 to 1.8 microns, even more preferably of about 1 micron.

Preferably, said step d) comprises:

> i. acidifying the aqueous phase of said step c) to a pH of less than 4, thus forming a precipitate comprising cannabinoid acids; even more preferably to a pH from 1 to 2.

> ii. separating the precipitate of step i. from the remaining aqueous phase, wherein said precipitate is said cannabinoid concentrate.

Preferably said step d) comprises:

> i. modifying the pH of the aqueous phase of said step c) to a value ranging from 12.5 to 9, thus forming a first precipitate;
> ii. separating the first precipitate of step i. from the remaining aqueous phase, wherein said first precipitate contains impurities, such as free fatty acids;
> iii. modifying the pH of the remaining aqueous phase of step ii. to a value of less than 12.5, thus forming a second precipitate;
> iv. separating the second precipitate from the further remaining aqueous phase, wherein said second precipitate is said cannabinoid concentrate.

Preferably, said step d) comprises drying the aqueous phase, thus forming a dried product, wherein said dried product is said cannabinoid concentrate.

Preferably, said cannabinoid concentrate comprises less than 0.3 %, more preferably less than 0.1 % by weight on total concentrate dry weight of total THC.

Preferably, said cannabinoid concentrate comprises less than 0.05 % by weight on total concentrate dry weight of total THC.

Preferably, said cannabinoid concentrate comprises at least 75% by weight, with respect to the total dry weight of the concentrate, of cannabinoids.

Preferably, said cannabinoid concentrate comprises at least 70 % by weight, with respect to the total dry weight of the concentrate, of cannabinoid acids other than THCA.

Preferably, said cannabinoid concentrate of step d) has a THCA content lower than that of the lipid extract of step a).

Preferably, said cannabinoid concentrate of step d) has a total THC lower than that of the lipid extract of step a).

Preferably, said step d) comprises drying, decarboxylating, distillating or crystallizing, said cannabinoid concentrate.

Thanks to the specific conditions of the method according to the invention, a cannabinoid concentrate is indeed obtained, showing an unexpectedly high level of preservation of cannabinoid acids other than THCA.

The method of the present invention produces a cannabinoid concentrate comprising more than 50% by weight, with respect to the total dry weight of the concentrate, of cannabinoid acids and not more than 0.3% by weight, with respect to The method of the present invention produces a cannabinoid concentrate comprising more than 50% by weight, with respect to the total dry weight of the concentrate, of cannabinoid acids and not more than 0.0001% per cent by weight of one or more organic solvents selected from a group consisting of acetone, benzene, butane, chloroform, cyclohexane, dichloromethane, ethanol, ethyl acetate, ethylbenzene, heptane, hexane, isobutane, isopropanol, methanol, pentane, propane, toluene, m-xylene, o-xylene, and p-xyleneheptane.

The method of the present invention produces a cannabinoid concentrate comprising more than 50% by weight, with respect to the total dry weight of the concentrate, of cannabinoids and less than 0.3% total THC.

The method of the present invention produces a cannabinoid concentrate comprising more than 50% by weight, with respect to the total dry weight of the concentrate, of cannabinoid acids and less than 0.3% total THC.

In a still further embodiment, the cannabinoid concentrate advantageously shows a THCA content with respect to the total dry weight of the concentrate lower than the THCA content of the starting biological material.

In a still further embodiment, the cannabinoid concentrate advantageously shows a THCA content with respect to the total dry weight of the concentrate lower than the THCA content of the lipid extract of step a).

In a still further embodiment, the cannabinoid concentrate advantageously shows a total THC content with respect to the total dry weight of the concentrate lower than the total THC content of the starting biological material and/or of the lipid extract.

The Applicant has noted that the combination of a high content of cannabinoid acids, combined with a particularly low content of THC and/or THCA and the absence or, at most, an extremely low content of organic solvents content is particularly surprising compared to the prior art concentrates, in which a high cannabinoid acids content is usually achieved by means of concentration or purification treatments that lead to an increase of THC and/or THCA or that involve the use of organic solvents, the elimination of which may result troublesome, expensive and not in compliance with regulatory requirements.

The other advantages of the cannabinoid concentrate have been disclosed in relation to the method according to the first aspect of the present invention and are not herewith repeated.

Preferably, said cannabinoid concentrate comprises more than 70% by weight, even more preferably more than 85% by weight, with respect to the total dry weight of the concentrate, of cannabinoid acids.

Preferably, the cannabinoid concentrate according to the invention comprises less than 0.1%, with respect to the total dry weight of the concentrate, of total THC.

Preferably, in the cannabinoid concentrate the cannabinoid acids are selected from the group consisting of: cannabidiolic acid (CBDA), cannabinolic acid (CBNA), cannabigerolic acid (CBGA), cannabichromenic acid (CBCA), cannabicyclolic acid (CBLA), cannabidivarinic acid (CBDVA), cannabigerovarinic acid (CBGVA), tetrahydrocanabivarinic acid (THCVA), cannabichromevarinic acid (CBCVA), cannabidiphorol acid (CBDPA) and Δ9-tetrahydrocannabiphorol acid (THCPA).

Preferably, in the cannabinoid concentrate the cannabinoid acids is CBDA. Preferably, in the cannabinoid concentrate according to the invention the cannabinoid acids is CBGA.

Thanks to its compositional and purity properties, said cannabinoid concentrate may be advantageously used for preparing pharmaceutical or nutraceutical products, cosmetics, food or feed products, antimicrobial, antibacterial, insecticidal or biopesticides containing one or more cannabinoids.

In a further aspect, therefore, the present invention relates to a method for preparing a pharmaceutical product, a nutraceutical product, a cosmetic product, a food product, a feed product, an antimicrobial, an antibacterial, an insecticide, a biopesticide, comprising the step of:

- providing a cannabinoid concentrate according to the present invention and/or preparing a cannabinoid concentrate according to the present invention; and
- obtaining a pharmaceutical product, a nutraceutical product, a cosmetic product, a food product, a feed product, an antimicrobial, an antibacterial, an insecticide, a biopesticide comprising one or more cannabinoids.

Further features and advantages of the invention will appear more clearly from the following description of some preferred embodiments thereof, made hereinafter by way of a non-limiting example with reference to the following exemplary examples.

## EXPERIMENTAL PART

### Example 1

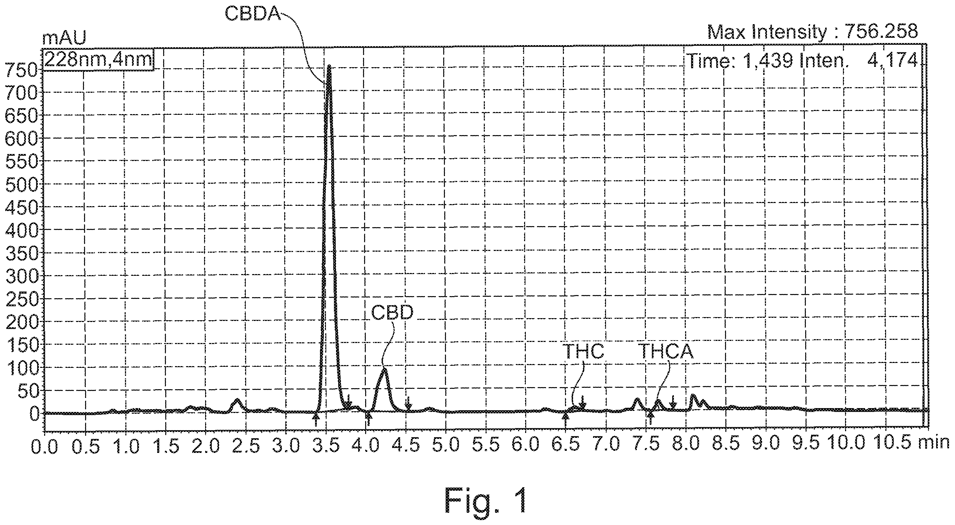

A refined sunflower oil based soluble extract ("Lipid extract") obtained according to Example 1 of WO 2018/130682, and having the composition reported in Table 1 as determined by HPLC analysis (Figure 1), was provided.

Table 1

| % by weight | THCA | THC | CBDA | CBD |
|---|---|---|---|---|
| Lipid extract | 0.06 | <0.05 | 3.10 | 0.80 |

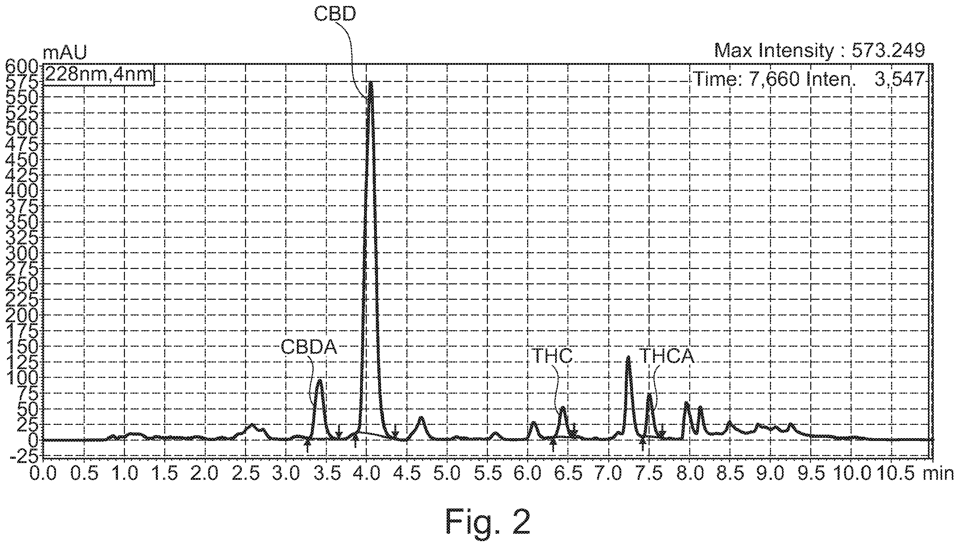

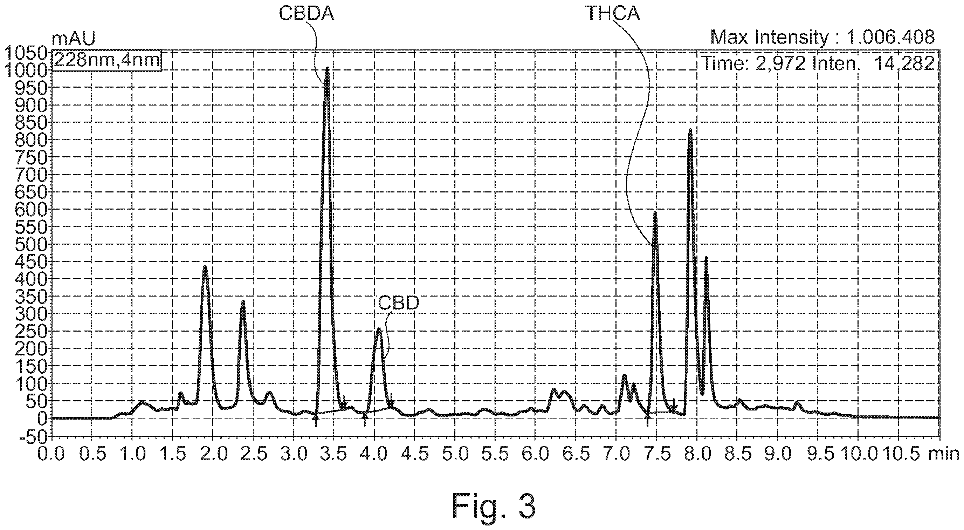

100 g of said extract were mixed with 480 g of an aqueous solution 0.355 M NaOH at room temperature in a kitchen robot Mulinex Companion, so to reach a pH of about 13. The mixture was kept in agitation for about 15 min. About 40 g NaCl were added to the mixture and mixed for 5 min before centrifugation. After mixture centrifugation (4.500 rpm for 10 min), 86 g of an oily lighter phase, 9 g of an intermediate semi-solid layer and 525 ml of a heavier aqueous phase were recovered. Samples of the lighter oily and intermediate layers were taken for HPLC cannabinoids analysis (respectively, Figure 2 and 3).

The heavier aqueous phase was filtered, utilizing a lab vacuum filter with a fiberglass filter having a pores diameter of about 1.6 micron. About 520 ml of filtered aqueous solution were recovered. A sample was taken and analysed for cannabinoids content.

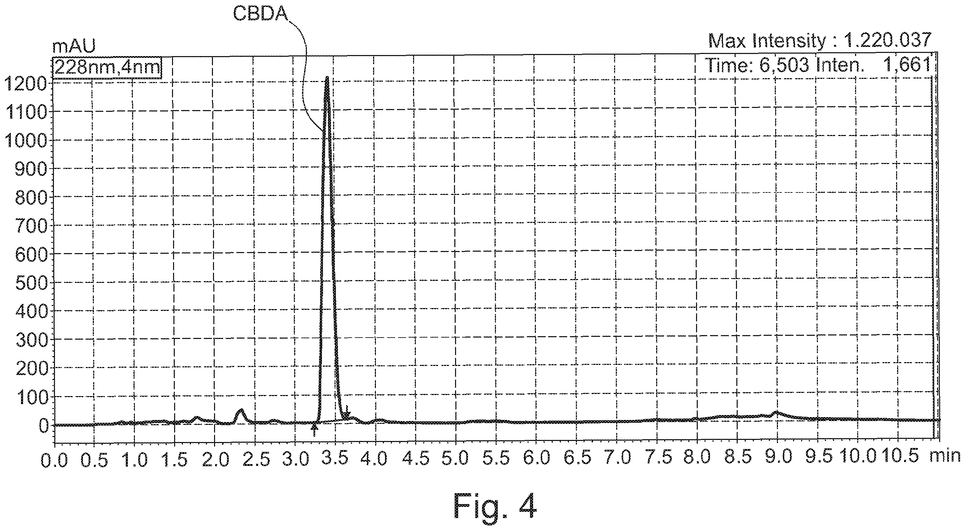

The filtered aqueous phase was acidified utilizing a solution of $H_3PO_4$ at 85% concentration to reach a pH of about 1.5-2 so to precipitate the cannabinoid acids. The cannabinoid acids were recovered utilizing a lab vacuum filter with fiberglass filter having pores diameter of 1.6 microns. After 24 hours air drying at room temperature, 3.06 g of cannabinoid precipitate were recovered. The cannabinoid precipitate was sampled and the sample sent out for HPLC analysis (Figure 4).

The quantitative determination of cannabinoids (CBD, CBDA, THCA) was performed by HPLC-DAD Shimadzu Nexera XR, equipped with a reverse phase C18 column NexLeaf CBX for Potency 150 x 4.6 mm, 2.7 $\mu$m.

Chromatographic conditions: Solvent A: water + 0.085% phosphoric acid (v/v); Solvent B: acetonitrile + 0.085% phosphoric acid (v/v). Flow: 1.6 ml/min. Oven temperature: 35 °C. Manual injection: loop 20 $\mu$L. Detection wavelength: 228 nm for CBD, THCA and THC; 307 nm for CBDA. Gradient elution: 70% of B up to 3 min, 85% of B to 7 min, 95% of B to 7.01 up to 8.00 min, and 70% of B up to 10 min.

Retention times: CBDA about 3.5 minutes; CBD about 4 minutes; CBN about 5.5 minutes; THC about 6.5 minutes; THCA about 7.5 minutes.

Figure 1 shows the HPLC trace of the lipid extract, Figure 2 shows the HPLC trace of the lighter oily phase, Figure 3 shows the HPLC trace of the intermediate slimy phase, and Figure 4 shows the HPLC trace of the precipitate obtained from the heavier aqueous phase thus obtained, in which the attribution of the chromatographic peaks of cannabinoids (CBDA, CBD, THC, THCA) is indicated.

The quantification of said cannabinoids was performed by an external standard method through the preparation of a calibration curve, using pure standard of cannabinoids (CBD, CBDA, THCA) in the range 2.5 - 250 ppm.

The THC quantification was done on the base of CBD calibration curve, using relative response factor (RRF) reported in the analytical monograph of the pharmacopeia (OMC / Farmalyse BV Version 7.1 / November 28, 2014).

The instrumental LCD was 0.5 ppm and the instrumental LOQ was 2.5 ppm.

LOD and LOQ values for the analytical method are reported, for each matrix, in the table below:

Table 2

| | | |
|---|---|---|
| Lipid extract | LOD | **0.0027%** |
| | LOQ | **0.0136%** |
| Lighter oily phase | LOD | **0.0027%** |
| | LOQ | **0.0136%** |
| Intermediate semi-solid layer | LOD | **0.0025%** |
| | LOQ | **0.0125%** |
| Filtered aqueous layer | LOD | **0.001%** |
| | LOQ | **0.005%** |
| Precipitate from aqueous phase | LOD | **0.005%** |
| | LOQ | **0.025%** |

The following cannabinoid concentrations (% w/w) in the cannabinoid various fractions obtained are reported:

Table 3

| % by dry weight | THCA | THC | CBDA | CBD |
|---|---|---|---|---|
| Lighter oily phase | 0.04 | < 0.05 | 0.06 | 0.78 |
| Intermediate phase | 0.30 | < LOD | 0.57 | 0.21 |
| Filtered aqueous phase | < LOD | < LOD | 81.34 | 1.98 |
| Precipitate from aqueous phase | < LOD | < LOD | 84.75 | 2.10 |

Considering cannabinoids extraction efficiency on different chemical forms, it was observed a surprising difference. While CBDA was recovered with an efficiency of about 84%, there was no THCA nor THC in the sample obtained from the filtered aqueous phase as well as in the precipitate obtained after acidifying the aqueous phase. THCA and THC mostly remained in the lighter oily phase.

The cannabinoid content of the precipitate was also remarkably high, about 85%, considering that no organic solvents were utilized.

This confirmed the effectiveness of the method according to the invention for recovering cannabinoids from a starting lipid extract without incurring in significant decarboxylation too.

**Example 2**

The same test as per example 1 was executed, with the only difference of adding and mixing for 30 min 5 g of calcium chloride in the aqueous phase before filtering.

About 2.66 g of precipitate were collected having a CBDA content of 88.2% and an undetectable level of THCA and THC. So calcium chloride helped in achieving a greater purity.

**Example 3**

The same test as per example 1 was executed, with the only difference of utilizing a liquid paraffine for obtaining the lipid extract.

Table 4

| % by weight | THCA | THC | CBDA | CBD |
|---|---|---|---|---|
| Lipid extract (paraffine) | 0.08 | 0.07 | 3.30 | 1.10 |

The following cannabinoid concentrations (% w/w) in the cannabinoid various fractions were obtained:

Table 5

| % by weight | THCA | THC | CBDA | CBD |
|---|---|---|---|---|
| Lighter oily phase | < LOD | 0.06 | < LOD | 0.93 |
| Intermediate phase | 0.25 | < LOD | 0.42 | 0,11 |
| Filtered and dried aqueous phase | < LOD | < LOD | 78.21 | 1.45 |
| Precipitate from aqueous phase | 0.02 | < LOD | 81.37 | 2.01 |

As it can be noticed, the cannabinoid acids were completely removed from the oily phase. On the other hand, some traces of THCA in the precipitate could still be measured.

**Example 4**

The same test as per example 1 was executed with the only difference of starting with a lipid extract containing CBGA.

Table 6

| % by weight | THCA | THC | CBGA | CBG |
|---|---|---|---|---|
| Lipid extract | 0.113 | < LOD | 9.45 | 0.12 |

The following cannabinoid concentrations (% w/w) in the cannabinoid various fractions were obtained:

Table 7

| % by weight | THCA | THC | CBGA | CBG |
|---|---|---|---|---|
| Lighter oily phase | 0.81 | < 0,05 | 0.97 | 0.09 |
| Intermediate phase | 0.08 | < LOD | 0.47 | 0,21 |

(continued)

| % by weight | THCA | THC | CBGA | CBG |
|---|---|---|---|---|
| Filtered and dried aqueous phase | < LOD | < LOD | 86,24 | 0,06 |
| Precipitate from aqueous phase | < LOD | < LOD | 89,88 | 0,10 |

As it can be noticed, a CBGA concentrate with undetectable level of THCA and THC was obtained.

**Example 5**

The same test as per example 1 was executed, with the only difference of acidifying the aqueous phase in two steps. In the first step the pH of the aqueous phase has been brought from about 13 to a pH of 12, utilizing a solution 1 M of H3PO4.

The aqueous phase has then been filtered to remove a first precipitate, utilizing a 1.6 microns fiberglass filter. The filtered solution has then been further acidified, utilizing a solution of H3PO4 at 85% concentration, to reach a pH of about 1.5-2 so to obtain a second precipitate. The first and second precipitate have been analyzed for cannabinoid content. While the first precipitate had a content of 2.1% CBD and 1.07 CBDA, the second precipitate had a content of 0.5% CBD and 90.5% CBDA.

**Example 6 (reference)**

An ethanolic lipophilic extract containing cannabinoids ("Ethanolic extract") was obtained according to the following steps:

a. mixing 150 g of Futura 75 hemp with 750 g of 99.9% pure ethanol at room temperature to form a mixture;
b. after stirring for 15 min, the mixture was separated via centrifugation (4.500 rpm for 10 min) into an ethanolic lighter cannabinoids-rich fraction and a heavier fraction containing spent biomass;
c. the lighter ethanolic fraction was kept at 4°C overnight to precipitate waxes (winterization) which were separated by filtration; the winterized ethanolic fraction was then sampled and analyzed for cannabinoid content with HPLC.

The winterized ethanolic extract was then mixed with 480 g of an aqueous solution 0.355 M NaOH at room temperature in a kitchen robot Mulinex Companion, so to reach a pH of about 13. The mixture was kept in agitation for about 15 min. About 10 g NaCl were added to the mixture and mixed for 5 min. The mixture was then filtered, utilizing a lab vacuum filter with a fiberglass filter having a pores diameter of about 1.6 micron. About 573 g of filtered solution were recovered. The filtered aqueous phase was acidified utilizing a solution of $H_3PO_4$ at 85% concentration to reach a pH of about 1.5-2, so to precipitate the cannabinoid acids. The cannabinoid acids were recovered utilizing a lab vacuum filter with fiberglass filter having pores diameter of 1.6 microns. After 24 hours air drying at room temperature, 2.1 g of cannabinoid precipitate were recovered. The cannabinoid precipitate was sampled and the sample sent out for HPLC analysis.

The cannabinoid concentrations (% w/w) in the ethanolic fraction and cannabinoid precipitate are reported in table 8:

Table **8**

| % by dry weight | THCA | THC | CBDA | CBD |
|---|---|---|---|---|
| Ethanolic lipophilic extract | 1.5 | 0.4 | 36,4 | 5.2 |
| Precipitate from aqueous phase | 2.7 | 0.1 | 71 | 0.4 |

As it can be noticed, applying the method according to the invention to an ethanolic lipophilic extract, CBDA and THCA were extracted and recovered with similar efficiencies and a concentrate with THCA content higher than the starting lipophilic extract was obtained. A concentrate with a total THC (THC + THCA) below the detection limit could not be obtained. This confirmed the effectiveness of the method according to the invention for recovering a cannabinoid concentrate from a lipid extract, wherein the cannabinoid concentrate has a total THC content lower than the initial lipid extract and preferably below the quantification level.

**Claims**

1. A method for preparing a cannabinoid concentrate comprising more than 50% by dry weight, with respect to the total dry weight of the concentrate, of cannabinoids and wherein the weight ratio (THCA):(other cannabinoid acids) is of less than 1:50, comprising the steps of:

   a) providing a lipid extract comprising at least 1% by dry weight, with respect to the total cannabinoids dry weight, of cannabinoid acids and wherein the weight ratio (THCA):(other cannabinoid acids) is of less than 1:2;
   b) mixing said lipid extract with an alkaline aqueous solution to form a mixture having a pH of at least 12;
   c) separating from the mixture of step b) an aqueous phase containing cannabinoid salts; and
   d) obtaining said cannabinoid concentrate,

   wherein before step c) at least one salt and/or at least one sugar is added to the mixture of step b), and said step c) of separating comprises separating from the mixture of step b):

   - a lighter oily phase,
   - an intermediate slimy phase, and
   - a heavier aqueous phase, wherein the heavier aqueous phase is the aqueous phase containing cannabinoid acid salts.

2. The method according to claim 1, wherein the alkaline aqueous solution of step b) comprises at least one hydroxide of at least one metal selected from the group consisting of: alkali metal, and alkaline earth metal.

3. The method according to claim 1 or 2, wherein in step b) the alkaline aqueous solution is added to the lipid extract in a weight ratio (alkaline aqueous solution):(lipid extract) of at least 2:1.

4. The method according to any one of claims 1-3, wherein the mixture of step b) the mixture of step b) is mixed at 25 °C for a time in the range from at least 5 seconds to less than 60 minutes.

5. The method according to any one of claims 1-4, wherein the mixture of step b) has a pH value ranging from 12.6 to 13.5.

6. The method accoding to any one of claims 1-5, wherein said step d) comprises:

   i. acidifying the aqueous phase of said step c) to a pH of less than 4, thus forming a precipitate comprising cannabinoid acids;
   ii. separating the precipitate of step i. from the remaining aqueous phase, wherein said precipitate is said cannabinoid concentrate.

7. The method according to anyone of claims 1-6, wherein said step d) comprises:

   i. modifying the pH of the aqueous phase of said step c) to a value ranging from 12.5 to 9, thus forming a first precipitate;
   ii. separating the first precipitate of step i. from the remaining aqueous phase, wherein said first precipitate contains impurities;
   iii. modifying the pH of the remaining aqueous phase of step ii. to a value of less than 12.5, thus forming a second precipitate;
   iv. separating the second precipitate from the further remaining aqueous phase, wherein said second precipitate is said cannabinoid concentrate.

8. The method according to any one of claims 1-5, wherein said step d) comprises drying the aqueous phase, thus forming a dried product, wherein said dried product is said cannabinoid concentrate.

9. The method according to any one of claims 1-8, wherein said cannabinoid concentrate comprises less than 0.3 %, by weight on total concentrate dry weight of total THC.

10. The method according to claim 9, wherein said cannabinoid concentrate comprises less than 0.1 %, by weight on total concentrate dry weight of total THC.

11. The method according to any one of claims 1-10, wherein said cannabinoid concentrate of step d) has a THCA content lower than that of the lipid extract of step a).

12. The method according to any one of claims 1-11, wherein said cannabinoid concentrate of step d) has a THCA content lower than that of the starting biological material.

13. The method according to any one of claims 1-12, wherein said cannabinoid concentrate of step d) has a total THC lower than that of the lipid extract of step a).

14. A method for preparing a pharmaceutical product, a nutraceutical product, a cosmetic product, a food product, a feed product, an antimicrobial, an antibacterial, an insecticide, a biopesticide, comprising the step of:

   - preparing a cannabinoid concentrate according to any one of claims 1-13; and
   - obtaining a pharmaceutical product, a nutraceutical product, a cosmetic product, a food product, a feed product, an antimicrobial, an antibacterial, an insecticide, a biopesticide comprising one or more cannabinoids.


**Patentansprüche**

1. Verfahren zur Herstellung eines Cannabinoid-Konzentrats, das mehr als 50% nach Trockengewicht, bezogen auf das gesamte Trockengewicht des Konzentrats, an Cannabinoiden umfasst, und wobei das Gewichtsverhältnis (THCA):(andere Cannabinoidsäuren) weniger als 1:50 beträgt, umfassend die Schritte:

   a) Bereitstellen eines Lipidextrakts, der mindestens 1% nach Trockengewicht, bezogen auf das Trockengewicht der gesamten Cannabinoide, an Cannabinoid-Säuren umfasst, und wobei das Gewichtsverhältnis (THCA):(andere Cannabinoidsäuren) weniger als 1:2 beträgt;
   b) Vermischen des Lipidextrakts mit einer alkalischen wässrigen Lösung, um ein Gemisch zu bilden, das einen pH von mindestens 12 aufweist;
   c) Abtrennen einer wässrigen Phase, die Cannabinoid-Salze enthält, von dem Gemisch von Schritt b); und
   d) Erhalten des Cannabinoid-Konzentrats,

   wobei vor Schritt c) mindestens ein Salz und/oder mindestens ein Zucker zu dem Gemisch von Schritt b) hinzugegeben wird, und der Schritt c) des Abtrennens ein Abtrennen von dem Gemisch von Schritt b):

   - einer leichteren, öligen Phase,
   - einer schleimigen Zwischenphase und
   - einer schwereren, wässrigen Phase umfasst, wobei die schwerere, wässrige Phase die wässrige Phase ist, die Cannabinoidsäuren-Salze enthält.

2. Verfahren nach Anspruch 1, wobei die alkalische wässrige Lösung von Schritt b) mindestens ein Hydroxid mindestens eines Metalls umfasst, das aus der Gruppe bestehend aus Alkalimetall und Erdalkalimetall ausgewählt ist.

3. Verfahren nach Anspruch 1 oder 2, wobei in Schritt b) die alkalische wässrige Lösung zu dem Lipidextrakt in einem Gewichtsverhältnis (alkalische wässrige Lösung):(Lipidextrakt) von mindestens 2:1 zugegeben wird.

4. Verfahren nach einem der Ansprüche 1 bis 3, wobei das Gemisch von Schritt b) das Gemisch von Schritt b) bei 25°C für eine Zeit im Bereich von mindestens 5 Sekunden bis weniger als 60 Minuten vermischt wird.

5. Verfahren nach einem der Ansprüche 1 bis 4, wobei das Gemisch von Schritt b) einen pH-Wert aufweist, der im Bereich von 12,6 bis 13,5 liegt.

6. Verfahren nach einem der Ansprüche 1 bis 5, wobei der Schritt d) umfasst:

   i. Ansäuern der wässrigen Phase von Schritt c) auf einen pH von weniger als 4, dadurch Bilden eines Niederschlags, der Cannabinoid-Säuren umfasst;
   ii. Abtrennen des Niederschlags von Schritt i. von der übrigen wässrigen Phase, wobei der Niederschlag das Cannabinoid-Konzentrat ist.

**7.** Verfahren nach einem der Ansprüche 1 bis 6, wobei der Schritt d) umfasst:

> i. Modifizieren des pH der wässrigen Phase von Schritt c) auf einen Wert, der im Bereich von 12,5 bis 9 liegt, dadurch Bilden eines ersten Niederschlags;
> ii. Abtrennen des ersten Niederschlags von Schritt i. von der übrigen wässrigen Phase, wobei der erste Niederschlag Verunreinigungen enthält;
> iii. Modifizieren des pH der übrigen wässrigen Phase von Schritt ii. auf einen Wert von weniger als 12,5, dadurch Bilden eines zweiten Niederschlags;
> iv. Abtrennen des zweiten Niederschlags von der weiteren übrigen wässrigen Phase, wobei der zweite Niederschlag das Cannabinoid-Konzentrat ist.

**8.** Verfahren nach einem der Ansprüche 1 bis 5, wobei der Schritt d) umfasst: Trocknen der wässrigen Phase, dadurch Bilden eines getrockneten Produkts, wobei das getrocknete Produkt das Cannabinoid-Konzentrat ist.

**9.** Verfahren nach einem der Ansprüche 1 bis 8, wobei das Cannabinoid-Konzentrat weniger als 0,3% nach Gewicht auf das gesamte Konzentrat-Trockengewicht an gesamtem THC umfasst.

**10.** Verfahren nach Anspruch 9, wobei das Cannabinoid-Konzentrat weniger als 0,1% nach Gewicht auf das gesamte Konzentrat-Trockengewicht an gesamtem THC umfasst.

**11.** Verfahren nach einem der Ansprüche 1 bis 10, wobei das Cannabinoid-Konzentrat von Schritt d) einen THCA-Gehalt aufweist, der niedriger ist als derjenige des Lipidextrakts von Schritt a).

**12.** Verfahren nach einem der Ansprüche 1 bis 11, wobei das Cannabinoid-Konzentrat von Schritt d) einen THCA-Gehalt aufweist, der niedriger ist als derjenige des biologischen Ausgangsmaterials.

**13.** Verfahren nach einem der Ansprüche 1 bis 12, wobei das Cannabinoid-Konzentrat von Schritt d) ein gesamtes THC aufweist, das niedriger ist als dasjenige des Lipidextrakts von Schritt a).

**14.** Verfahren zur Herstellung eines pharmazeutischen Produkts, eines nutrazeutischen Produkts, eines kosmetischen Produkts, eines Nahrungsmittelprodukts, eines Futterprodukts, eines Antimikrobiotikums, eines Antibiotikums, eines Insektizids, eines Biopestizids, das den Schritt umfasst:

> - Herstellen eines Cannabinoid-Konzentrats nach einem der Ansprüche 1 bis 13; und
> - Erhalten eines pharmazeutischen Produkts, eines nutrazeutischen Produkts, eines kosmetischen Produkts, eines Nahrungsmittelprodukts, eines Futterprodukts, eines Antimikrobiotikums, eines Antibiotikums, eines Insektizids, eines Biopestizids, das ein oder mehrere Cannabinoide umfasst.

**Revendications**

**1.** Procédé de préparation d'un concentré de cannabinoïdes comprenant plus de 50 % en poids sec, par rapport au poids sec total du concentré, de cannabinoïdes et dans lequel le rapport pondéral (THCA) : (autres acides cannabinoïdes) est inférieur à 1:50, comprenant les étapes consistant à :

> a) fournir un extrait lipidique comprenant au moins 1 % en poids sec, par rapport au poids sec total des cannabinoïdes, d'acides cannabinoïdes et dans lequel le rapport pondéral (THCA) : (autres acides cannabinoïdes) est inférieur à 1:2 ;
> b) mélanger ledit extrait lipidique avec une solution aqueuse alcaline pour former un mélange présentant un pH d'au moins 12 ;
> c) séparer du mélange de l'étape b) une phase aqueuse contenant des sels de cannabinoïdes ; et
> d) obtenir ledit concentré de cannabinoïdes,

dans lequel avant l'étape c) au moins un sel et/ou au moins un sucre est ajouté au mélange de l'étape b), et ladite étape c) de séparation comprend la séparation du mélange de l'étape b) de:

> - une phase huileuse plus légère,
> - une phase visqueuse intermédiaire, et

- une phase aqueuse plus lourde, dans lequel la phase aqueuse plus lourde est la phase aqueuse contenant des sels d'acide cannabinoïde.

2. Procédé selon la revendication 1, dans lequel la solution aqueuse alcaline de l'étape b) comprend au moins un hydroxyde d'au moins un métal choisi dans le groupe constitué par : un métal alcalin et un métal alcalino-terreux.

3. Procédé selon la revendication 1 ou 2, dans lequel dans l'étape b) la solution aqueuse alcaline est ajoutée à l'extrait lipidique dans un rapport pondéral (solution aqueuse alcaline):(extrait lipidique) d'au moins 2:1.

4. Procédé selon l'une quelconque des revendications 1 à 3, dans lequel le mélange de l'étape b) le mélange de l'étape b) est mélangé à 25 °C pendant une durée comprise entre au moins 5 secondes et moins de 60 minutes.

5. Procédé selon l'une quelconque des revendications 1 à 4, dans lequel le mélange de l'étape b) présente une valeur de pH comprise entre 12,6 et 13,5.

6. Procédé selon l'une quelconque des revendications 1 à 5, dans lequel ladite étape d) comprend :

    i. acidifier la phase aqueuse de ladite étape c) à un pH inférieur à 4, formant ainsi un précipité comprenant des acides cannabinoïdes ;
    ii. séparer le précipité de l'étape i. de la phase aqueuse restante, dans lequel ledit précipité est ledit concentré de cannabinoïdes.

7. Procédé selon l'une quelconque des revendications 1 à 6, dans lequel ladite étape d) comprend :

    i. modifier le pH de la phase aqueuse de ladite étape c) à une valeur comprise entre 12,5 et 9, formant ainsi un premier précipité ;
    ii. séparer le premier précipité de l'étape i. de la phase aqueuse restante, dans lequel ledit premier précipité contient des impuretés ;
    iii. modifier le pH de la phase aqueuse restante de l'étape ii. à une valeur inférieure à 12,5, formant ainsi un deuxième précipité ;
    iv. séparer le deuxième précipité de l'autre phase aqueuse restante, dans lequel ledit deuxième précipité est ledit concentré de cannabinoïde.

8. Procédé selon l'une quelconque des revendications 1 à 5, dans lequel ladite étape d) comprend le séchage de la phase aqueuse, formant ainsi un produit séché, dans lequel ledit produit séché est ledit concentré de cannabinoïde.

9. Procédé selon l'une quelconque des revendications 1 à 8, dans lequel ledit concentré de cannabinoïdes comprend moins de 0,3 %, en poids par rapport au poids sec total du concentré de THC total.

10. Procédé selon la revendication 9, dans lequel ledit concentré de cannabinoïdes comprend moins de 0,1 %, en poids par rapport au poids sec total du concentré de THC total.

11. Procédé selon l'une quelconque des revendications 1 à 10, dans lequel ledit concentré de cannabinoïdes de l'étape d) présente une teneur en THCA inférieure à celle de l'extrait lipidique de l'étape a).

12. Procédé selon l'une quelconque des revendications 1 à 11, dans lequel ledit concentré de cannabinoïdes de l'étape d) présente une teneur en THCA inférieure à celle de la matière biologique de départ.

13. Procédé selon l'une quelconque des revendications 1 à 12, dans lequel ledit concentré de cannabinoïdes de l'étape d) présente une teneur en THCA inférieure à celle de l'extrait lipidique de l'étape a).

14. Procédé de préparation d'un produit pharmaceutique, d'un produit nutraceutique, d'un produit cosmétique, d'un produit alimentaire, d'un produit alimentaire pour animaux, d'un antimicrobien, d'un antibactérien, d'un insecticide, d'un biopesticide, comprenant l'étape consistant à :

    - préparer un concentré de cannabinoïdes selon l'une quelconque des revendications 1 à 13 ; et
    - obtenir un produit pharmaceutique, un produit nutraceutique, un produit cosmétique, un produit alimentaire, un produit alimentaire pour animaux, un antimicrobien, un antibactérien, un insecticide, un biopesticide comprenant un ou plusieurs cannabinoïdes.

Fig. 1

EP 4 208 274 B1

Fig. 2

EP 4 208 274 B1

Fig. 3

EP 4 208 274 B1

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017025712 A1
- WO 2019012267 A
- US 9186386 B2
- US 6403126 B1
- WO 2019057994 A1
- US 2019359550 A
- US 7592468 B2
- US 9340475 B2
- WO 2004026857 A2
- WO 2020028991 A
- WO 2018130682 A